# EUROPEAN PATENT APPLICATION

(11) **EP 0 843 006 A2**
(43) Date of publication of application: **20.05.1998**
(21) Application number: 97309108.5
(22) Date of filing: 12.11.1997
(51) Int. Cl.: C12N 5/10, C12Q 1/37, C12N 15/57, C12N 9/64

(54) **Recombinant PSA expression vectors and assays using the same**

(30) Priority: 15.11.1996 US 30947 P
(71) Applicant: ELI LILLY AND COMPANY, Indianapolis, Indiana 46285 (US)
(72) Inventor: Dixon, Eric Paul, Apex, North Carolina 27502 (US); Little, Sheila Parks, Indianapolis, Indiana 46208 (US); Sutkowski, Debra Mary, Lawrenceville, New Jersey 08648 (US)
(74) Representative: Denholm, Anna Marie

(57) **Abstract**

The present invention provides novel recombinant DNA expression vectors that encode prostate specific antigen or modified prostate specific antigen. The vectors allow for the expression of prostate specific antigen in an active form in human prostate cell lines transformed with these novel vectors. The transformed host cells express prostate specific antigen or precursors, derivatives, or subfragments thereof in an active form. The present invention also provides in vivo and in vitro assays for determining those compounds which modulate PSA.

## Description

Prostatic cancer has become the most commonly diagnosed cancer and the second leading cause of cancer death among males in the United States [Cancer Facts & Figures, pp 10-11 (1995)]. In order to study the biologic behavior of prostate tumor growth and to effectively test new therapeutic approaches, a suitable cell line which produces enzymatically active PSA is necessary.

PSA is an androgen-regulated, kallikrein-like serine proteinase expressed and implicated in mediating growth of prostate, bone and breast tissues [J. Clin. Endoc. Metab. **73**:401-7 (1991); Biochem. Biophys. Res. Commun. **192**:940-7 (1993); Cancer Res. 54:6344-7 (1994)]. Elevated PSA levels in the circulation are associated with the pathogenesis of benign or malignant proliferative disorders of the prostate [New Engl. J. Med. **317**:909-6 (1987); New Engl. J. Med. **324**:1156-61 (1991)]. Data from a variety of cell culture systems support the contention that the addition of PSA to the medium is associated with mitogenic responses *in vitro*. A variety of mechanisms have been proposed to mediate these proliferative responses. PSA has been shown to cleave Insulin-like growth factor binding protein-3 (IGFBP3) to decrease its affinity for Insulin-like growth factor-l (IGF-I) [J. Clin. Endoc. Metab. **75**:1046-53 (1992)]. Dissociation of the IGF-I--IGFBP3 complex renders IGF-I available for binding to its plasma membrane receptor which stimulates proliferation of normal [J. Clin. Endoc. Metab. **73**:401-7 (1991)] and neoplastic human prostatic epithelial as well as human BPH stromal cells. An in vivo correlation also exists between PSA and IGFBP's in the serum of patients with prostatic cancer [J. Clin. Endoc. Metab. **77**:229-33 (1993)]. Elevated serum PSA correlates with high levels of serum IGFBP2 and reduced levels of serum IGFBP3 compared to healthy male controls. This observation supports the pathophysiological significance of the observation by Cohen and associates that IGFBP3 is an *in vivo* substrate for PSA [J. Clin. Endoc. Metab. **75**:1046-53 (1992)].

PSA is highly abundant in human seminal fluid (0.5-2.0 mg/ml) [World J. Urol. **11**:188-91 (1993)] and may be responsible for proteolytically generating inhibin-like and other protein growth factors from semenogelin, the primary protein constituent of semen. The primary biologic role of PSA is cleavage of semenogelin I, semenogelin 11 and fibronectin, resulting in increased sperm motility [J. Clin. Invest. **80**:281-5 (1987)]. Parathyroid hormone-related protein (PTHrP), which is present in the ejaculate, has been associated with fetal development [J. Pathol. **167**:291-6 (1992)] and the transformed phenotype in some adult tissues [J. Clin. Invest. **83**:1057-60 (1989)]. PTHrP is expressed by prostatic neuroendocrine cells and prostatic cancer cells [Urology **43**:667-74 (1994)] and has been reported to be a putative substrate for PSA, the most abundant proteinase in seminal fluid [J. Urol. **153**(4) Abstract #1555 (1995)]. PTHrP has also been implicated as a potential autocrine growth regulator in human prostatic cancer cell lines [Urol. **43**:675-9 (1994)]. Recent studies demonstrate that PSA can antagonize parathyroid hormone-stimulated bone resorption and enhance bone formation through liberation of free IGF-I. PSA has been shown to have a direct stimulatory effect on the rat osteoblast cell line, UMR106 [Biochem. Biophys. Res. Commun. **192**:940-7 (1993)]. Addition of PSA to the media caused a significant increase in UMR106 cell growth. Part of this stimulation was attributed to the activation of latent TGF-b by PSA. The above data demonstrates that enzymatically active PSA can cleave several substrates which are involved in growth regulation.

Measurement of circulating serum PSA has found widespread use in monitoring patients with prostate cancer, as well as in the early detection of prostate cancer.

Treatment of early prostate cancer in men under the age of 65 has focused on radical surgery and/or radiotherapy, but the impact of these aggressive approaches on overall survival remains debatable.

In addition to medical castration and/or radiation therapy, chemical castration and anti-androgens with and without castration have been used as treatment protocols. These treatments, while in certain instances are effective, do not completely irradicate the disease.

It would be advantageous to find additional non-invasive means such as pharmaceutical compounds for arresting the growth of prostate cancer cells that can either be used alone or in conjunction with other treatments.

Accordingly, there exists a need for producing stably transfected cell lines which produce PSA in an active form. Such cell lines could then be used in an assay to rapidly and effectively test for modulators of PSA. To date, there does not exist a stably transfected human prostate cancer cell line which expresses proteolytically active PSA.

It has now been found that certain human prostate cell lines can be stably transfected with a DNA expression vector encoding proteolytically active PSA.

The present invention provides novel recombinant DNA expression vectors that encode prostate specific antigen or modified prostate specific antigen. The vectors allow for the expression of prostate specific antigen in an active form in human prostate cell lines transformed with these novel vectors. The transformed host cells express prostate specific antigen or precursors, derivatives, or subfragments thereof in an active form. The present invention also provides in vivo and in vitro assays for determining compounds which modulate PSA.

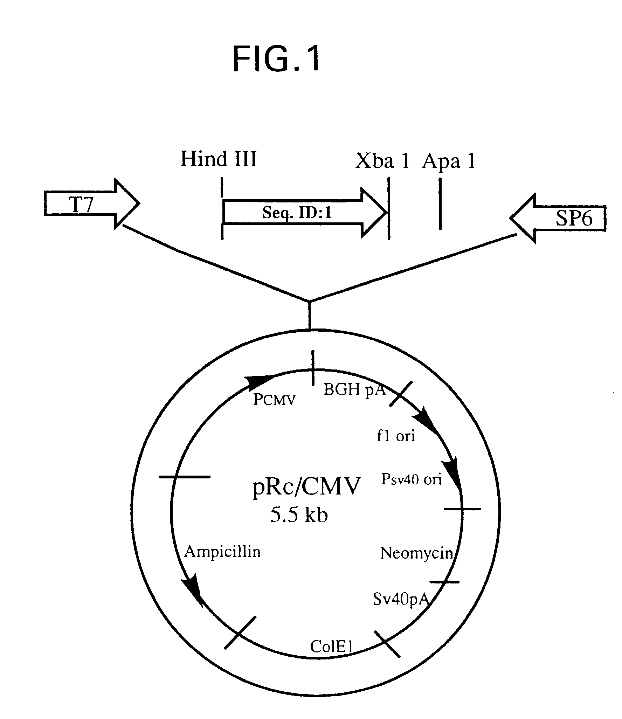

Figure 1 is a restriction and function map of the plasmid pRc/CMV-PSA·WT which contains nucleotides 13-826 of SEQ ID NO:1.

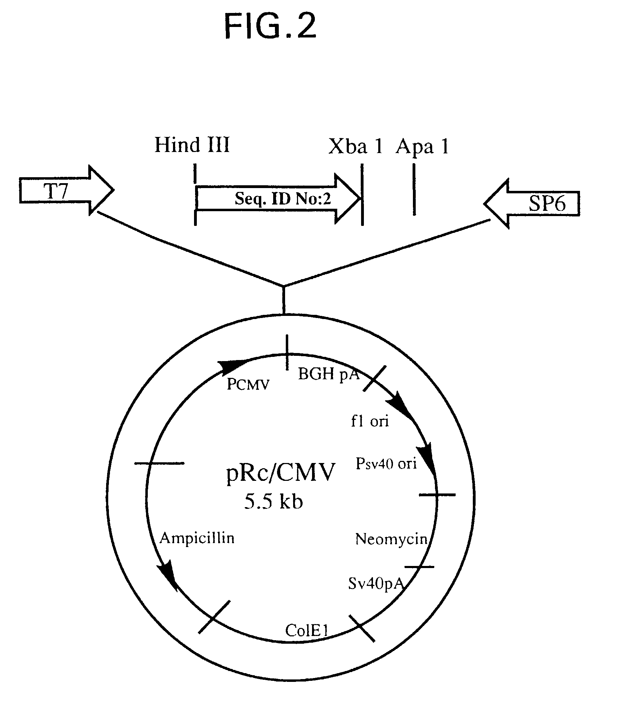

Figure 2 is a restriction and function map of the plasmid pRc/CMV-PSA·EPRO which contains nucleotides 13-805 of SEQ ID NO:2.

The terms and abbreviations used in this document have their normal meanings unless otherwise designated.

All nucleic acid sequences, unless otherwise designated, are written in the direction from the 5' end to the 3' end, frequently referred to as "5' to 3'".

All amino acid or protein sequences, unless otherwise designated, are written commencing with the amino terminus ("N-terminus") and concluding with the carboxy terminus ("C-terminus").

"Base pair" or "bp" as used herein refers to DNA or RNA. The abbreviations A,C,G, and T correspond to the 5'-monophosphate forms of the deoxyribonucleosides (deoxy) adenosine, (deoxy) cytidine, (deoxy)guanosine, and (deoxy)thymidine, respectively, when they occur in DNA molecules. The abbreviations U,C,G, and A correspond to the 5'-monophosphate forms of the ribonucleosides urodine, cytidine, guanosine, and adenosine, respectively when they occur in RNA molecules. In double stranded DNA, base pair may refer to a pairing of A with T or C with G. In a DNA/ RNA, heteroduplex base pair may refer to a pairing of A with U or C with G. (See definition of "complementary", infra.).

The terms "cleavage" or "restriction" of DNA refers to the catalytic cleavage of the DNA with a restriction enzyme that acts only at certain sequences in the DNA ("sequence-specific endonucleases"). The various restriction enzymes used herein are commercially available and their reaction conditions, cofactors, and other requirements were used as would be known to one of ordinary skill in the art. Appropriate buffers and substrate amounts for particular restriction enzymes are specified by the manufacturer or can be readily found in the literature.

"Ligation" refers to the process of forming phosphodiester bonds between two nucleic acid fragments (T. Maniatis, et al., supra., p. 146). Unless otherwise provided, ligation may be accomplished using known buffers and conditions with a DNA ligase, such as T4 DNA ligase.

The term "plasmid" refers to an extrachromosomal (usually) self-replicating genetic element. Plasmids are generally designated by a lower case "p" followed by letters and/or numbers. The starting plasmids herein are either commercially available, publicly available on an unrestricted basis, or can be constructed from available plasmids in accordance with published procedures. In addition, equivalent plasmids to those described are known in the art and will be apparent to the ordinarily skilled artisan.

"Recombinant DNA cloning vector" as used herein refers to any autonomously replicating agent, including, but not limited to, plasmids and phages, comprising a DNA molecule to which one or more additional DNA segments can or have been added.

The term "recombinant DNA expression vector" as used herein refers to any recombinant DNA cloning vector in which a promoter and other regulatory elements to control transcription of the inserted DNA.

The term "expression vector system" as used herein refers to a recombinant DNA expression vector in combination with one or more trans-acting factors that specifically influence transcription, stability, or replication of the recombinant DNA expression vector. The transacting factor may be expressed from a co-transfected plasmid, virus, or other extrachromosomal element, or may be expressed from a gene integrated within the chromosome.

"Transcription" as used herein refers to the process whereby information contained in a nucleotide sequence of DNA is transferred to a complementary RNA sequence.

The term "transfection" as used herein refers to the taking up of an expression vector by a host cell whether or not any coding sequences are in fact expressed. Numerous methods of transfection are known to the ordinarily skilled artisan, for example, calcium phosphate co-precipitation, and electroporation. Successful transfection is generally recognized when any indication of the operation of this vector occurs within the host cell.

The term "transformation" as used herein means the introduction of DNA into an organism so that the DNA is replicable, either as an extrachromosomal element or by chromosomal integration. Methods of transforming bacterial and eukaryotic hosts are well known in the art, many of which methods are summarized in J. Sambrook, et al., "Molecular Cloning: A Laboratory Manual" (1989).

The term "translation" as used herein refers to the process whereby the genetic information of messenger RNA is used to specify and direct the synthesis of a polypeptide chain.

The term "vector" as used herein refers to a nucleic acid compound used for the transformation of cells with polynucleotide sequences corresponding to appropriate protein molecules which when combined with appropriate control sequences confer specific properties on the host cell to be transformed. Plasmids, viruses, and bacteriophage are suitable vectors. Artificial vectors are constructed by joining DNA molecules from different sources. The term "vector" as used herein includes Recombinant DNA cloning vectors and Recombinant DNA expression vectors.

The terms "complementary" or "complementarity" as used herein refers to the pairing of bases, purines and pyrimidines, that associate through hydrogen bonding in double stranded nucleic acid. The following base pairs are complementary: guanine and cytosine; adenine and thymine; and adenine and uracil.

The term "hybridization" as used herein refers to a process in which a strand of nucleic acid joins with a complementary strand through base pairing. The conditions employed in the hybridization of two non-identical, but very similar, complementary nucleic acids varies with the degree of complementarity of the two strands and the length of the strands. Such techniques and conditions are well known to practitioners in this field.

A "primer" is a nucleic acid fragment which functions as an initiating substrate for enzymatic or synthetic elongation.

The term "promoter" refers to a DNA sequence which directs transcription of DNA to RNA.

A "probe" as used herein is a nucleic acid compound or a fragment thereof which hybridizes with a nucleic acid compound which encodes either the entire sequence for PSA, a sequence complementary to the sequence for PSA, or a part thereof.

The term "PCR" as used herein refers to the widely-known polymerase chain reaction employing a thermally-stable polymerase.

The term "PSA" as used herein, refers to prostate specific antigen.

The present invention provides a variety of novel recombinant DNA expression vectors that comprise the DNA sequence for PSA or a modified sequence for PSA. These vectors allow for the effective production of the proteolytically active PSA protein or modified PSA protein in stably transfected host cells. This in turn provides an effective means to assay for possible modulators of PSA.

The present invention provides a DNA expression vector encoding the isolated nucleic acid compound having the sequence: 
AGCCCCAAGC TTACCACCTG CACCCGGAGA GCTGTGTGTC ACCATGTGGG 50

TCCCGGTTGT CTTCCTCACC CTGTCCGTGA CGTGGATTGG TGCTGCACCC 100

CTCATCCTGT CTCGGATTGT GGGAGGCTGG GAGTGCGAGA AGCATTCCCA 150

ACCCTGGCAG GTGCTTGTGG CCTCTCGTGG CAGGGCAGTC TGCGGCGGTG 200

TTCTGGTGCA CCCCCAGTGG GTCCTCACAG CTGCCCACTG CATCAGGAAC 250

AAAAGCGTGA TCTTGCTGGG TCGGCACAGC CTGTTTCATC CTGAAGACAC 300

AGGCCAGGTA TTTCAGGTCA GCCACAGCTT CCCACACCCG CTCTACGATA 350

TGAGCCTCCT GAAGAATCGA TTCCTCAGGC CAGGTGATGA CTCCAGCCAC 400

GACCTCATGC TGCTCCGCCT GTCAGAGCCT GCCGAGCTCA CGGATGCTGT 450

GAAGGTCATG GACCTGCCCA CCCAGGAGCC AGCACTGGGG ACCACCTGCT 500

ACGCCTCAGG CTGGGGCAGC ATTGAACCAG AGGAGTTCTT GACCCCAAAG 550

AAACTTCAGT GTGTGGACCT CCATGTTATT TCCAATGACG TGTGTGCGCA 600

AGTTCACCCT CAGAAGGTGA CCAAGTTCAT GCTGTGTGCT GGACGCTGGA 650

CAGGGGGCAA AAGCACCTGC TCGGGTGATT CTGGGGGCCC ACTTGTCTGT 700

AATGGTGTGC TTCAAGGTAT CACGTCATGG GGCAGTGAAC CATGTGCCCT 750

GCCCGAAAGG CCTTCCCTGT ACACCAAGGT GGTGCATTAC CGGAAGTGGA 800

TCAAGGACAC CATCGTGGCC AACCCCTGAG CACCCCTATC AACCCCCTAT 850

TGTAGTAAAC TTGGAACCTT GGAAATGACC AGGCCAAGAC TCAAGCCTCC 900

CCAGTTCTAC TGACCTTTGT CCTTAGGTGT GAGGTCCAGG GTTGCTAGGA 950

AAAGAAATCA GCAGACACAG GTGTAGACCA GAGTGTTTCT TAAATGGTGT 1000

AATTTTGTCC TCTCTGTGTC CTGGGGAATA CTGGCCATGC CTGGAGACAT 1050
 
ATCACTCAAT TTCTCTGAGG ACACAGATAG GATGGGGTGT CTGTGTTATT 1100

TGTGGGGTAC AGAGATGAAA GAGGGGTGGG ATCCACACTG AGAGAGTGGA 1150

GAGTGACATG TGCTGGACAC TGTCCATGAA GCACTGAGCA GAAGCTGGAG 1200

GCACAACGCA CCAGACACTC ACAGCAAGGA TGGAGCTGAA AACATAACCC 1250

ACTCTGTCCT GGAGGCACTG GGAAGCCTAG AGAAGGCTGT GAGCCAAGGA 1300

GGGAGGGTCT TCCTTTGGCA TGGGATGGGG ATGAAGTAAG GAGAGGGACT 1350

GGACCCCCTG GAAGCTGATT CACTATGGGG GGAGGTGTAT TGAAGTCCTC 1400

CAGACAACCC TCAGATTTGA TGATTTCCTA GTAGAACTCA CAGAAATAAA 1450

GAGCTGTTAT ACTGTG 1466
 which is hereinafter designated as SEQ ID NO:1. More particularily, nucleotides 13-826 of SEQ ID NO:1 are preferred to prepared the expression vectors of the present invention.

In an alternative embodiment, the DNA expression vectors of the present invention comprise expression vectors which encode an isolated nucleic acid compound having the sequence: 
AGCCCCAAGC TTACCACCTG CACCCGGAGA GCTGTGTGTC ACCATGTGGG 50
TCCCGGTTGT CTTCCTCACC CTGTCCGTGA CGTGGATTGG TGCTATTGTG 100
GGAGGCTGGG AGTGCGAGAA GCATTCCCAA CCCTGGCAGG TGCTTGTGGC 150
CTCTCGTGGC AGGGCAGTCT GCGGCGGTGT TCTGGTGCAC CCCCAGTGGG 200
TCCTCACAGC TGCCCACTGC ATCAGGAACA AAAGCGTGAT CTTGCTGGGT 250
CGGCACAGCC TGTTTCATCC TGAAGACACA GGCCAGGTAT TTCAGGTCAG 300
CCACAGCTTC CCACACCCGC TCTACGATAT GAGCCTCCTG AAGAATCGAT 350
TCCTCAGGCC AGGTGATGAC TCCAGCCACG ACCTCATGCT GCTCCGCCTG 400
TCAGAGCCTG CCGAGCTCAC GGATGCTGTG AAGGTCATGG ACCTGCCCAC 450
CCAGGAGCCA GCACTGGGGA CCACCTGCTA CGCCTCAGGC TGGGGCAGCA 500
TTGAACCAGA GGAGTTCTTG ACCCCAAAGA AACTTCAGTG TGTGGACCTC 550
 
CATGTTATTT CCAATGACGT GTGTGCGCAA GTTCACCCTC AGAAGGTGAC 600

CAAGTTCATG CTGTGTGCTG GACGCTGGAC AGGGGGCAAA AGCACCTGCT 650

CGGGTGATTC TGGGGGCCCA CTTGTCTGTA ATGGTGTGCT TCAAGGTATC 700

ACGTCATGGG GCAGTGAACC ATGTGCCCTG CCCGAAAGGC CTTCCCTGTA 750

CACCAAGGTG GTGCATTACC GGAAGTGGAT CAAGGACACC ATCGTGGCCA 800

ACCCCTGAGC ACCCCTATCA ACCCCCTATT GTAGTAAACT TGGAACCTTG 850

GAAATGACCA GGCCAAGACT CAAGCCTCCC CAGTTCTACT GACCTTTGTC 900

CTTAGGTGTG AGGTCCAGGG TTGCTAGGAA AAGAAATCAG CAGACACAGG 950

TGTAGACCAG AGTGTTTCTT AAATGGTGTA ATTTTGTCCT CTCTGTGTCC 1000

TGGGGAATAC TGGCCATGCC TGGAGACATA TCACTCAATT TCTCTGAGGA 1050

CACAGATAGG ATGGGGTGTC TGTGTTATTT GTGGGGTACA GAGATGAAAG 1100

AGGGGTGGGA TCCACACTGA GAGAGTGGAG AGTGACATGT GCTGGACACT 1150

GTCCATGAAG CACTGAGCAG AAGCTGGAGG CACAACGCAC CAGACACTCA 1200

CAGCAAGGAT GGAGCTGAAA ACATAACCCA CTCTGTCCTG GAGGCACTGG 1250

GAAGCCTAGA GAAGGCTGTG AGCCAAGGAG GGAGGGTCTT CCTTTGGCAT 1300

GGGATGGGGA TGAAGTAAGG AGAGGGACTG GACCCCCTGG AAGCTGATTC 1350

ACTATGGGGG GAGGTGTATT GAAGTCCTCC AGACAACCCT CAGATTTGAT 1400

GATTTCCTAG TAGAACTCAC AGAAATAAAG AGCTGTTATA CTGTG 1445
 which is hereinafter designated as SEQ ID NO:2. More particularily, nucleotides 13-805 of SEQ ID NO:2 are preferred to prepared the expression vectors of the present invention.

The DNA compounds utilized in the present invention were derived from reverse transcribed mRNA from human prostate. See specifically, FEBS Lett., **214**:317-22 (1987). In one embodiment, a clone encoding the full sequence for PSA, including the signal peptide and propeptide portion, was used (SEQ ID NO:1). In an alternative embodiment, PSA cDNA was manipulated to construct a DNA molecule which did not include the propeptide portion of the sequence for PSA (SEQ ID NO:2). The two cDNA containing plasmids were designated pCMV-PSA·WT and pCMV-PSA·ÆPRO, respectively.

While the DNA compounds of the present invention were prepared by the method noted above, DNA encoding the protein of interest can be constructed by any of the methods known in the art, including, but not limited to the isolation of natural product, synthetic or semisynthetic processes. Alternatively, the desired DNA sequences can be generated using the polymerase chain reaction as described in U.S. Patent No. 4,889,818, which is herein incorporated by reference.

The DNA compound is integrated into an expression vector in a manner suitable for the expression of the protein of interest. The skilled artisan understands that the type of cloning vector or expression vector employed typically depends upon the availability of appropriate restriction sites, the type of host cell in which the vector is to be transfected or transformed, the purpose of the transfection or transformation, the presence or absence of readily assayable markers, and the number of copies of the gene to be present in the cell.

The vector utilized in the present invention is the pRc/CMV eucaryotic expression vector (obtained from Invitrogen). pRc/CMV is a eukaryotic expression vector designed for high level stable expression. The vector includes the beta-lactamase gene for ampicillin selection in procaryotes, a high copy origin of replication from pVC19, the m13 origin of replication, T7 and SP6 promotors for sense and anti-sense transcription and a multiple cloning site. DNA inserted into the multiple cloning sites are expressed from the first AUG codon following the CMV (cytomegalovirus) promotor and enhancer. The neomycin resistance gene is expressed from the SV40 early promotor. The vector also includes the bovine growth hormone polyadenylation signal for polyadenylation of mRNA's.

The present DNA recombinant expression vectors are designed to transform mammalian host cells. A variety of host cells are commercially available from suppliers such as: Bethesda Research Laboratories, Gaithersburg, Maryland 20877 and Stratagene Cloning Systems, La Jolla, California 92037; or are readily available to the public from sources such as the American Type Culture Collection, 12301 Parklawn Drive, Rockville, Maryland, 10852-1776.

The pRc/CMV·PSA plasmids of the present invention function most efficiently when introduced into a prostate cancer cell line. A variety of prostate cancer host cell lines are available from the American Type Culture Collection (ATTC) including, DU145 (isolated from a brain metastatic lesion), LnCaP (isolated from a lymph node metastatic lesion), and PC-3 (isolated from a bone metastatic lesion).

According to one embodiment of the present invention, PC-3 host cells are selected to serve as host cells for the expression of PSA. The PSA DNA is inserted into a pRc/CMV expression vector which is suitable to achieve mammalian cell expression of the DNA, a vector which exploits the promotor of the virus that infects human prostate cell lines. The DNA is inserted into the vector at a site downstream from the CMV promotor. Specifically, HindIII and XbaI restriction sites located downstream of the promotor are digested to allow insertion of a DNA strand, which includes PSA encoding DNA, the ends of which are appropriate for ligation to the HindIII and Xbal enzyme-digested ends of the vector. The DNA is incorporated into the pRc/CMV vector by ligating the appropriate cDNA portion into the vector and then transfecting the host cell by electroporation as described in the Examples. The ratio of vector to cDNA insert used in the present invention was 10ng vector:100ng of insert.

Transfection of the prostate cancer cells with the vectors of the present invention was performed by electroporation. See, e.g., J. Sambrook, et al., supra, at 3:16.30-3:16.66. Following transfection the cells are typically incubated for a brief period in nonselective medium and are then transferred to selective medium and observed for proliferation. After a sufficient time for cell outgrowth, the supernatants are screened for the presence of PSA protein. Stable cell lines expressing active PSA were clonally selected.

Cells stably transformed with PSA-containing vector are grown in culture media and under growth conditions to facilitate the growth of the particular host cell used. One of skill in the art would be familiar with the media and other growth conditions required by the particular host cell chosen, as such information is well-documented in the art.

The present invention is not limited to the expression of the particular PSA derivatives disclosed. The present DNA compounds are readily modified to delete certain other portions encoding various amino acid residues of PSA. Those skilled in the art recognize that restriction enzyme digestion or site-directed mutagenesis upon the DNA compounds of the present invention will yield an almost limitless group of molecules which will encode for PSA derivatives. Such manipulations are within the scope of this invention, and can be performed given the detailed sequences disclosed herein.

Skilled artisans also recognize that some alterations of the PSA sequences will fail to change the function of the amino acid compound encoded by the PSA sequences. Many modifications and variations of the present illustrative DNA sequences and plasmids are possible.

Alterations of the protein may be induced by alterations of the nucleic acid compounds which encodes these proteins. These mutations of the nucleic acid compound may be generated by either random mutagenesis techniques, such as those techniques employing chemical mutagens, or by site-specific mutagenesis employing oligonucleotides. Those nucleic acid compounds which confer substantially the same function in substantially the same manner as the exemplified nucleic acid compounds are also encompassed within the present invention.

The synthetic PSA gene may be designed to possess restriction endonuclease cleavage sites at either end of the transcript to facilitate isolation from and integration into expression and amplification plasmids. The choice of restriction sites are chosen so as to properly orient the coding sequence of the receptor with control sequences to achieve proper in-frame reading and expression of the PSA receptor molecule. A variety of other such cleavage sites may be incorporated depending on the particular plasmid constructs employed and may be generated by techniques well known in the art.

Those skilled in the art will recognize that the expression vectors of this invention are used to transform eukaryotic host cells, particularily prostate cancer cell lines, such that a polypeptide with the PSA structure is expressed by the host cell. By transforming host cells that possess the cellular machinery with which to process the signal peptide, with a vector comprising a promoter that functions in the host cell and drives transcription of the PSA structural gene, mature PSA can be found within the cell.

Determination of the ability of compounds to stimulate or inhibit the proteolytic activity of PSA is essential to further development of such compounds for therapeutic applications. The need for a bioactivity assay system which determines the response of PSA to a compound is clear. The instant invention provides such a bioactivity assay, said assay comprising the steps of:
a) transfecting a prostate cancer host cell with an expression vector comprising DNA encoding PSA or modified PSA;
b) culturing said host cell under conditions such that the PSA or modified PSA protein is expressed;
c) exposing said host cell so transfected to a test compound; and
d) measuring the change in proteolytic activity of PSA relative to a control in which the transfected host cell is not exposed to a test compound.

Active PSA produced by recombinant methodology will have a profound effect upon the diagnosis, prognosis, treatment and study of prostate cancer. The transfected cell lines disclosed can be used to assay for inhibitors of PSA. In addition to in vitro assays, the transfected cell lines may be used in in vivo studies. For example, such cell lines can be introduced either subcutaneously or onto the clavaria of normal mice or rats to determine if metastasis of tumor cells is induced by the presence of PSA.

The following examples more fully describe the present invention. Those skilled in the art will recognize that the particular reagents, equipment, and procedures described in the Examples are merely illustrative and are not intended to limit the present invention in any manner.

### EXAMPLES

### I. cDNA Encoding Human Prostate Specific Antigen Constructs:

### A. PSA-WT (wildtype) Construct

PSA cDNA was isolated from human prostate mRNA, which was reverse transcribed with Superscript II (BRL), then PCR amplified using Taq DNA polymerase and oligoucleotide primers based on the cDNA sequence reported for human prostate specific antigen. [FEBS Lett. **214**(2):317-22 (1987)]. To obtain wild type PSA, two primers: 
PSA-5′ sense primer:  5′-ACCACCTGCACCCGGAGAAGC-3′ (SEQ ID
NO:3); and
 
PSA-3′ antisense primer:  5′-CACAGTATAACAGCTCTTTATTTC-3′
(SEQ ID NO:4)
 were used to amplify a 1.45 kb product. This 1.45 kb product was purified from an agarose gel using Prep-a-gene (Bio-Rad). To facilitate directional cloning of PSA into the expression vector pRc/CMV, 5' and 3' sequences encoding unique restriction endonuclease sites were added to the PCR primers. A 20X excess of HindIII and NotI were added to the purified PCR product, then digested overnight at 37°C to create sticky ends for ligation into pRc/CMV. The molar ratio of insert to vector was greater then 20:1. Ligated ampicillin resistant cDNAs were then transformed into Rec A⁻ E. coli and plasmid cDNA was isolated using standard alkaline lysis methods.

### B. PSA-ÆPRO (propeptide deleted) Construct

The PSA construct in which the propeptide portion is deleted was generated by PCR using two primers: 
PSA-5′ sense primer and PSA-2 antisense 5′-
GCACTCCCAGCCTCCCACAATAGCACCAATCCACGTCACGGA-3′ (SEQ ID
NO:5); and PSA-3 antisense primer and PSA-3 5′-
TCCGTGACGTGGATTGGTGCTATTGTGGGAGGCTGGGAGTGC-3′ (SEQ ID
NO:6).
 The PCR products were gel purified as described then denatured at 100_C. Reannealing occurred by slow cooling to 4_C. DNA was extended using Klenow polymerase and dNTP mixtures. The full length deleted pro-PSA was further amplified using PSA-5' and PSA-3' primers,then gel purified [Biotechniques **14**(3):366-9 (Mar. 1993)].

### II. Construction of the Plasmids

### A. Construction of the plasmid CMV·PSA-WT

The 1.45 kb product was ligated into HindIII/XbaI sites located within the polylinker of pRc/CMV vector. The ligation mixture was transformed into DH10B cells following BRL protocols, plated onto TY-amp agar dishes, then grown overnight at 37_C. Restriction enzyme digest analysis and primary sequence analysis of the subcloned cDNA fragments confirmed that the clones contained the PSA gene.

### B. Construction of the plasmid RC/CMV·PSA-ÆPRO

The full length PSA-ÆPRO product was ligated into the pRc/CMV vector according to the procedure noted above for pCMV·PSA-WT.

### III. Transfection of Cell Lines

### A. Cell Lines

The parental PC-3 (CRI 1435) and DU-145 (HTB 81) human prostatic adenocarcinoma cell lines were purchased from ATCC. These cell lines were maintained in RPMI-1640 media (Gibco-BRL) supplemented with 10% Fetal Bovine Serum (FBS).

### B. Electroporation of Transfected Cell Lines

Stable expression of PSA plasmid DNA (pRc/CMV-PSA·WT) into PC-3 and DU-145 cells was achieved via electroporation using a Gene Pulsar apparatus (Bio-Rad) at a capacitance setting of 960 microfarad and voltage setting of 0.4 kV [Nucleic Acids Res. **15**(3):1311-1326 (1987)]. The following procedure was performed at 4_C or on ice. The cells were washed 1X in PBS and collected by centrifugation for 5 minutes at 2000 x g. PC-3 cells (5 x 10⁶), sonicated salmon sperm DNA (20 µg), and plasmid DNA (25 µg) were electroporated in a pre-chilled 0.4 cm gap cuvette (Bio-Rad). The cells were immediately transferred to cold RPMI-1640 media supplemented with 10% FBS, then plated onto 100mm x 20mm dishes. After 48 hours, this media was exchanged with RPMI-1640/10% FBS containing 500mg/ml G418 sulfate to select for clones containing the PSA expression plasmid. After two weeks the concentration of G418 was increased to 1 mg/ml and individual colonies were isolated and cultured for enzymatic activity. Four weeks after electroporation, individual colonies were harvested and plated onto 6-well culture dishes and subcloned.

Stable expression of PSA plasmid DNA (pRc/CMV-PSA·ÆPRO) into PC-3 cells was achieved in the same manner as noted above for pRc/CMV-PSA·WT.

Expression and secretion of PSA by DU145 cells was achieved in the same manner as noted above for PC-3 cells. Higher activity and stability was obtained in the PC-3 cell line.

### C. Cell Extracts and Western Blot Analysis

Transfected cells, (PC-3 with pRc/CMV-PSA·WT), were lysed in PBS using a cell disrupter. Equal volumes of cell lysate or conditioned media was added to tricine SDS sample buffer, boiled for 5 minutes and electrophoresed on a Tris-Glycine 4-20% acrylamide gel in SDS denaturing buffer. Gels were electroblotted to PVDF membranes (Bio-Rad) for 2 hours at 100 mA in Tris-Glycine transfer buffer. Blots were probed with a rabbit polyclonal anti-PSA at 1:3000. Immunopositive bands were then detected for each cell line using ECL chemiluminescense (Amersham) thereby indicating that PSA was being produced by transfected cell line.

### IV. Colormetric Assay for Active PSA Production

Conditioned media from PSA-producing PC-3 clones (pRc/CMV-PSA·WT) were evaluated for enzymatic activity using a substrate-protease colorimetric assay which is based on fractionation of a synthetic peptide. The activity of the PSA in the conditioned media was based on the concurrent testing of known concentration of purified, enzymatically active PSA from seminal plasma. PC-3 cells transfected with pRc/CMV-PSA·WT were allowed to grow in T150 tissue culture flasks in serum-free media. After 120 hours, the conditioned media was collected, centrifuged to remove cellular debris and concentrated from 375ml to 10ml. This concentrated sample was then tested for PSA activity.

Two different PBS-BSA Buffers were used. Buffer A comprised PBS + 0.7 mg BSA/ml and was used for dilution of the PSA control. Buffer B comprised PBS + 6.3 mg BSA/ml and was used in the reaction mixture to prevent non-specific binding to the microtiter plate. Both buffers were stored at 4°C. Disposable, non-sterile, polystyrene (not treated), 96 well, flat-bottom, microtiter assay plates from Corning (Catalog #25880-96) were used. A Bio-Tek Instruments, Inc. Microtiter Plate Reader, CERES UV900HDi, was used to read the colormetric changes. Dual wavelength (reading at 405 nm with a reference wavelength of 450 nm) was utilized when reading wells.

The chymotrypsin tripeptide substrate (MeO-Suc-Arg-Pro-Tyr-pNA) was obtained from Pharmacia Hepar Chromogenix [a chromogenic substrate for chymotrypsin; Catalog # S-2586 (25 mg vials); molecular weight =705.3; stored at room temperature (kept dry) until reconstituted; reconstituted in PBS at 10 mM (7.05 mg/ml) and stored at 4° C as described by the product insert]. The PBS used was Gibco BRL Dulbecco's Phosphate-Buffered Saline 1X (D-PBS) (Catalog No. 14040-026) stored at 4° C. The BSA (Bovine Serum Albumin) was obtained from Sigma (Catalog No. #A-7511, stored at 4° C.).

PBS/BSA solutions, and the substrate solution (3546 ml PBS/25 mg substrate) were removed from the refrigerator and warmed to 37°C for at least 30 minutes. The outer wells of the plates were not used. All samples were run in duplicate. At least one well was designated as an experimental blank (hereinafter "BLANK well"). At least one positive control which utilized PSA having a known activity was also established (hereinafter "POSITIVE well"). Test sample(s) were included in all other well (hereinafter "TEST wells").

10 µl of PBS buffer B was added to all reaction BLANK wells, POSITIVE wells and TEST wells followed by 10 µl of DMSO in each well.

10 µl of stock PSA was diluted with 610 µl PBS Buffer A. 10 µl of PBS + 0.7 mg BSA/ml was added to each BLANK well. 10 µl of diluted PSA (40 nM final concentration) was added to each POSITIVE wells. 10 µl of the concentrated culture was added to each TEST wells. The microtiter plate was gently shaken (manually) to ensure adequate mixing of well contents, wells were sealed with parafilm and preincubated for 2 hours at 37°C. Following pre-incubation, 70 µl of substrate stock solution (1 mM final concentration) was added to all wells. Reaction progress, PSA cleavage of p-nitroaniline (p-NA) from the substrate resulting in appearance of yellow color, was monitored for the ensuing 10 minutes. Twenty optical density readings were automatically taken and stored by the instrument.

Following completion of data accumulation, printouts of the reaction profile in all wells was obtained. From this data, it was determined that PC-3 transfected cell lines were producing active PSA. PSA activity of the transfected PC-3 cell line was measured at 27.5 ng/ml in media conditioned over a 120 hour incubation period

## Claims

1. A transfected prostate cancer host cell harboring a recombinant DNA expression vector which is pRc/CMV said recombinant DNA expression vector comprising the nucleic acid compound of SEQ ID NO:1 in combination with regulatory elements necessary for expression of the nucleic acid compound in the host cell.

2. The transfected host cell of Claim 1 wherein the nucleic acid compound comprises nucleotides 13 through 826 of SEQ ID NO:1.

3. The transfected host cell of Claim 1 wherein the DNA expression vector is plasmid pRc/CMV-PSA·WT.

4. The transfected host cell of Claim 3 wherein the host cell is PC-3.

5. A transfected prostate cancer host cell harboring a recombinant DNA expression vector which is pRc/CMV said recombinant DNA expression vector comprising the nucleic acid compound of SEQ ID NO:2 in combination with regulatory elements necessary for expression of the nucleic acid compound in the host cell.

6. The transfected host cell of Claim 5 wherein the nucleic acid compound comprises nucleotides 13 through 805 of SEQ ID NO:2.

7. The transfected host cell of Claim 5 wherein the DNA expression vector is plasmid pRc/CMV-PSA·ÆPRO.

8. The transfected host cell of Claim 8 wherein the host cell is PC-3.

9. A method of evaluating the effectiveness of a test compound for the treatment or prevention of a condition associated with an increase in prostate specific antigen which method comprises the steps of:
a) transfecting a prostate cancer host cell with an expression vector comprising DNA encoding PSA or modified PSA;
b) culturing said host cell under conditions such that the PSA or modified PSA protein is expressed;
c) exposing said host cell so transfected to a test compound; and
d) measuring the change in a proteolytic activity of
PSA relative to a control in which the transfected host cell is not exposed to a test compound.


SEQUENCE LISTING

(1) GENERAL INFORMATION:

(i) APPLICANT: ELI LILLY AND COMPANY
(B) STREET: Lilly Corporate Center
(C) CITY: Indianapolis
(D) STATE: Indiana
(E) COUNTRY: United States of America
(F) ZIP: 46285

(ii) TITLE OF INVENTION: Recombiant PSA Expression Vectors and
Assays Using the Same

(iii) NUMBER OF SEQUENCES: 6

(iv) CORRESPONDENCE ADDRESS:
(A) ADDRESSEE: Dr. A. M. Denholm
(B) STREET: Erl Wood Manor
(C) CITY: Windlesham
(D) STATE: Surrey
(E) COUNTRY: United Kingdom
(F) ZIP: GU20 6PH

(v) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30

(2) INFORMATION FOR SEQ ID NO:1:

(i) SEQUENCE CHARACTERISTICS:
(A) LENGTH: 1466 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(xi) SEQUENCE DESCRIPTION: SEQ ID NO:1:

AGCCCCAAGC TTACCACCTG CACCCGGAGA GCTGTGTGTC ACCATGTGGG TCCCGGTTGT 60

CTTCCTCACC CTGTCCGTGA CGTGGATTGG TGCTGCACCC CTCATCCTGT CTCGGATTGT 120

GGGAGGCTGG GAGTGCGAGA AGCATTCCCA ACCCTGGCAG GTGCTTGTGG CCTCTCGTGG 180

CAGGGCAGTC TGCGGCGGTG TTCTGGTGCA CCCCCAGTGG GTCCTCACAG CTGCCCACTG 240

CATCAGGAAC AAAAGCGTGA TCTTGCTGGG TCGGCACAGC CTGTTTCATC CTGAAGACAC 300

AGGCCAGGTA TTTCAGGTCA GCCACAGCTT CCCACACCCG CTCTACGATA TGAGCCTCCT 360

GAAGAATCGA TTCCTCAGGC CAGGTGATGA CTCCAGCCAC GACCTCATGC TGCTCCGCCT 420

GTCAGAGCCT GCCGAGCTCA CGGATGCTGT GAAGGTCATG GACCTGCCCA CCCAGGAGCC 480

AGCACTGGGG ACCACCTGCT ACGCCTCAGG CTGGGGCAGC ATTGAACCAG AGGAGTTCTT 540

GACCCCAAAG AAACTTCAGT GTGTGGACCT CCATGTTATT TCCAATGACG TGTGTGCGCA 600

AGTTCACCCT CAGAAGGTGA CCAAGTTCAT GCTGTGTGCT GGACGCTGGA CAGGGGGCAA 660

AAGCACCTGC TCGGGTGATT CTGGGGGCCC ACTTGTCTGT AATGGTGTGC TTCAAGGTAT 720

CACGTCATGG GGCAGTGAAC CATGTGCCCT GCCCGAAAGG CCTTCCCTGT ACACCAAGGT 780

GGTGCATTAC CGGAAGTGGA TCAAGGACAC CATCGTGGCC AACCCCTGAG CACCCCTATC 840

AACCCCCTAT TGTAGTAAAC TTGGAACCTT GGAAATGACC AGGCCAAGAC TCAAGCCTCC 900

CCAGTTCTAC TGACCTTTGT CCTTAGGTGT GAGGTCCAGG GTTGCTAGGA AAAGAAATCA 960

GCAGACACAG GTGTAGACCA GAGTGTTTCT TAAATGGTGT AATTTTGTCC TCTCTGTGTC 1020

CTGGGGAATA CTGGCCATGC CTGGAGACAT ATCACTCAAT TTCTCTGAGG ACACAGATAG 1080

GATGGGGTGT CTGTGTTATT TGTGGGGTAC AGAGATGAAA GAGGGGTGGG ATCCACACTG 1140

AGAGAGTGGA GAGTGACATG TGCTGGACAC TGTCCATGAA GCACTGAGCA GAAGCTGGAG 1200

GCACAACGCA CCAGACACTC ACAGCAAGGA TGGAGCTGAA AACATAACCC ACTCTGTCCT 1260

GGAGGCACTG GGAAGCCTAG AGAAGGCTGT GAGCCAAGGA GGGAGGGTCT TCCTTTGGCA 1320

TGGGATGGGG ATGAAGTAAG GAGAGGGACT GGACCCCCTG GAAGCTGATT CACTATGGGG 1380

GGAGGTGTAT TGAAGTCCTC CAGACAACCC TCAGATTTGA TGATTTCCTA GTAGAACTCA 1440

CAGAAATAAA GAGCTGTTAT ACTGTG 1466

(2) INFORMATION FOR SEQ ID NO:2:

(i) SEQUENCE CHARACTERISTICS:
(A) LENGTH: 1445 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(xi) SEQUENCE DESCRIPTION: SEQ ID NO:2:

AGCCCCAAGC TTACCACCTG CACCCGGAGA GCTGTGTGTC ACCATGTGGG TCCCGGTTGT 60

CTTCCTCACC CTGTCCGTGA CGTGGATTGG TGCTATTGTG GGAGGCTGGG AGTGCGAGAA 120

GCATTCCCAA CCCTGGCAGG TGCTTGTGGC CTCTCGTGGC AGGGCAGTCT GCGGCGGTGT 180

TCTGGTGCAC CCCCAGTGGG TCCTCACAGC TGCCCACTGC ATCAGGAACA AAAGCGTGAT 240

CTTGCTGGGT CGGCACAGCC TGTTTCATCC TGAAGACACA GGCCAGGTAT TTCAGGTCAG 300

CCACAGCTTC CCACACCCGC TCTACGATAT GAGCCTCCTG AAGAATCGAT TCCTCAGGCC 360

AGGTGATGAC TCCAGCCACG ACCTCATGCT GCTCCGCCTG TCAGAGCCTG CCGAGCTCAC 420

GGATGCTGTG AAGGTCATGG ACCTGCCCAC CCAGGAGCCA GCACTGGGGA CCACCTGCTA 480

CGCCTCAGGC TGGGGCAGCA TTGAACCAGA GGAGTTCTTG ACCCCAAAGA AACTTCAGTG 540

TGTGGACCTC CATGTTATTT CCAATGACGT GTGTGCGCAA GTTCACCCTC AGAAGGTGAC 600

CAAGTTCATG CTGTGTGCTG GACGCTGGAC AGGGGGCAAA AGCACCTGCT CGGGTGATTC 660

TGGGGGCCCA CTTGTCTGTA ATGGTGTGCT TCAAGGTATC ACGTCATGGG GCAGTGAACC 720

ATGTGCCCTG CCCGAAAGGC CTTCCCTGTA CACCAAGGTG GTGCATTACC GGAAGTGGAT 780

CAAGGACACC ATCGTGGCCA ACCCCTGAGC ACCCCTATCA ACCCCCTATT GTAGTAAACT 840

TGGAACCTTG GAAATGACCA GGCCAAGACT CAAGCCTCCC CAGTTCTACT GACCTTTGTC 900

CTTAGGTGTG AGGTCCAGGG TTGCTAGGAA AAGAAATCAG CAGACACAGG TGTAGACCAG 960

AGTGTTTCTT AAATGGTGTA ATTTTGTCCT CTCTGTGTCC TGGGGAATAC TGGCCATGCC 1020

TGGAGACATA TCACTCAATT TCTCTGAGGA CACAGATAGG ATGGGGTGTC TGTGTTATTT 1080

GTGGGGTACA GAGATGAAAG AGGGGTGGGA TCCACACTGA GAGAGTGGAG AGTGACATGT 1140

GCTGGACACT GTCCATGAAG CACTGAGCAG AAGCTGGAGG CACAACGCAC CAGACACTCA 1200

CAGCAAGGAT GGAGCTGAAA ACATAACCCA CTCTGTCCTG GAGGCACTGG GAAGCCTAGA 1260

GAAGGCTGTG AGCCAAGGAG GGAGGGTCTT CCTTTGGCAT GGGATGGGGA TGAAGTAAGG 1320

AGAGGGACTG GACCCCCTGG AAGCTGATTC ACTATGGGGG GAGGTGTATT GAAGTCCTCC 1380

AGACAACCCT CAGATTTGAT GATTTCCTAG TAGAACTCAC AGAAATAAAG AGCTGTTATA 1440

CTGTG 1445

(2) INFORMATION FOR SEQ ID NO:3:

(i) SEQUENCE CHARACTERISTICS:
(A) LENGTH: 21 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(xi) SEQUENCE DESCRIPTION: SEQ ID NO:3:

ACCACCTGCA CCCGGAGAAG C 21

(2) INFORMATION FOR SEQ ID NO:4:

(i) SEQUENCE CHARACTERISTICS:
(A) LENGTH: 24 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(xi) SEQUENCE DESCRIPTION: SEQ ID NO:4:

CACAGTATAA CAGCTCTTTA TTTC 24

(2) INFORMATION FOR SEQ ID NO:5:

(i) SEQUENCE CHARACTERISTICS:
(A) LENGTH: 42 base pairs

(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(xi) SEQUENCE DESCRIPTION: SEQ ID NO:5:

GCACTCCCAG CCTCCCACAA TAGCACCAAT CCACGTCACG GA 42

(2) INFORMATION FOR SEQ ID NO:6:

(i) SEQUENCE CHARACTERISTICS:
(A) LENGTH: 42 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(xi) SEQUENCE DESCRIPTION: SEQ ID NO:6:

TCCGTGACGT GGATTGGTGC TATTGTGGGA GGCTGGGAGT GC 42